# FASCICULE DE BREVET EUROPEEN

(11) **EP 2 146 954 B1**
(45) Date de publication et mention de la délivrance du brevet: **31.10.2012**
(21) Numéro de dépôt: 08788207.2
(22) Date de dépôt: 18.04.2008
(51) Int. Cl.: C07C 273/18, C07C 309/65, C07C 309/66, C07C 309/73, C07C 69/734, C07C 275/42

(54) **PROCEDE DE SYNTHESE DE L'ACIDE (Z)-3-[2-BUTOXY-3'-(3-HEPTYL-1-METHYL-UREIDO)-BIPHENYL-4-YL]-2-METHOXY-ACRYLIQUE**
VERFAHREN ZUR SYNTHESE VON (Z)-3-[2-BUTOXY-3'-(3-HEPTYL-1-METHYLUREID)-BIPHENYL-4-YL]-2-METHOXY-ACRYLSÄURE
METHOD FOR THE SYNTHESIS OF (Z)-3-[2-BUTOXY-3'-(3-HEPTYL-1-METHYL-UREIDO)-BIPHENYL-4-YL]-2-METHOXY-ACRYLIC ACID

(30) Priorité: 11.05.2007 FR 0755021
(43) Date de publication de la demande: 27.01.2010
(73) Titulaire: Galderma Research & Development, 06410 Biot (FR)
(72) Inventeur: BOITEAU, Jean-Guy, 34130 Saint-Aunes (FR)
(74) Mandataire: Boulard, Denis
(86) Numéro de dépôt international: PCT/FR2008/050706
(87) Numéro de publication internationale: WO 2008/139121

(56) Documents cités:
- WO-A-2007/049158
- SUZUKI A: "Recent advances in the cross-coupling reactions of organoboron derivatives with organic electrophiles,1995-1998" JOURNAL OF ORGANOMETALLIC CHEMISTRY, ELSEVIER-SEQUOIA S.A. LAUSANNE, CH, vol. 576, 1999, pages 147-168, XP002206661 ISSN: 0022-328X
- ISHIYAMA T ET AL: "SYNTHESIS OF ARYLBORONATES VIA THE PALLADIUM(0)-CATALYZED CROSS- COUPLING REACTION OF TETRA(ALKOXO)DIBORONS WITH ARYL TRIFLATES" TETRAHEDRON LETTERS, ELSEVIER, AMSTERDAM, NL, vol. 38, no. 19, 12 mai 1997 (1997-05-12), pages 3447-3450, XP000686267 ISSN: 0040-4039
- S.L.BUCHWALD: "The First General Palladium Catalyst for the Suzuki-Miyaura and Carbonyl Enolate Coupling of Aryl Arenesulfonates" JACS, no. 125, 2003, pages 11818-11819, XP002462170

## Description

La présente invention est relative à un nouveau procédé de synthèse de l'acide (Z)-3-[2-Butoxy-3'-(3-heptyl-1-methyl-ureido)-biphenyl-4-yl]-2-methoxy-acrylique de formule (I), 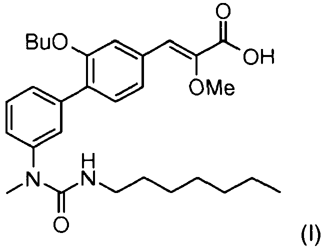 via l'intermédiaire réactionnel de formule générale (XII),

| | | | |
|---|---|---|---|
| R2 = | CF3 | (XIIa) | 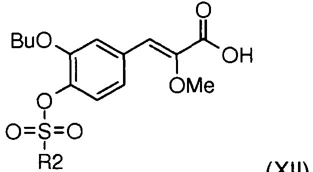 |
| | CH3 | (XIIb) | |
| | Ph | (XIIc) | |
| | 4-Tolyl | (XIId) | (XII) |

L'acte (Z)-3-[2-Butoxy-3'-(3-heptyl-1-methyl-ureido)-biphenyl-4-yl]-2-methoxy-acrylique de formule (1) est un modulateur des récepteurs PPARs (de l'anglais peroxisome proliferating activated receptors).

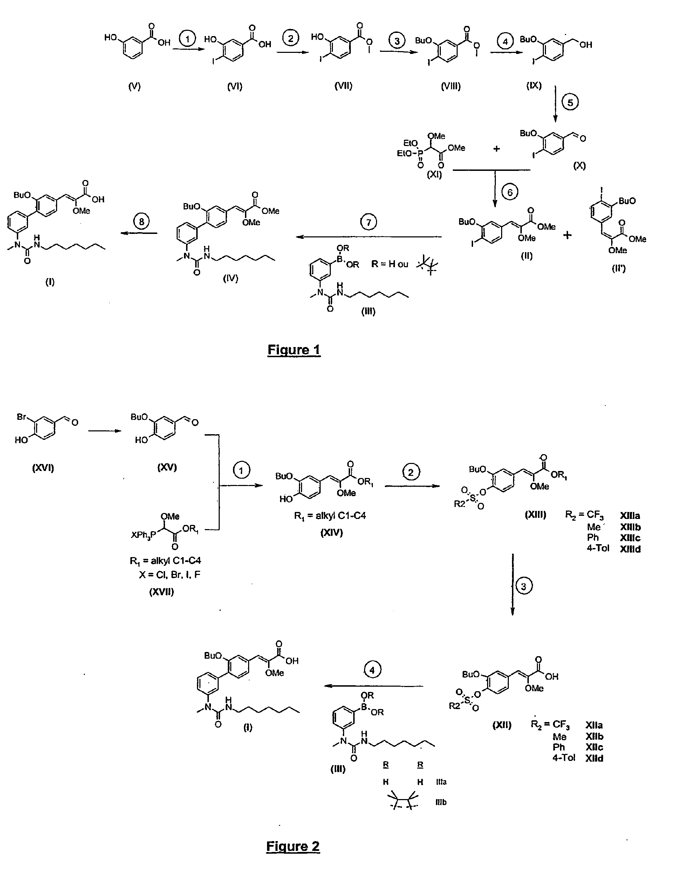

La synthèse du composé de formule (1) a été décrite en 8 étapes dans la demande internationale WO2007/04915 et telle que présentée à la figure 1 :

Etape 1 : L'acide 3-hydroxy-benzoique (V) est iodé en présence d'iodure de sodium et d'hypochlorite de sodium pour donner l'acide 4-iodo-3-hydroxy-benzoique (VI).

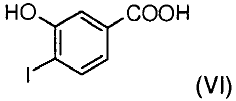

Etape 2 : L'acide 4-iodo-3-hydroxybenzoique (VI) est estérifié avec du méthanol en présence d'acide sulfurique pour fournir le 3-hydroxy-4-iodo-benzoate de méthyle (VII).

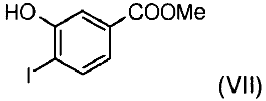

Etape 3 : La réaction entre le 3-hydroxy-4-iodo-benzoate de méthyle (VII) et l'iodure de butyle en présence de carbonate de potassium permet d'obtenir le 3-butoxy-4-iodobenzoate de methyle (VIII).

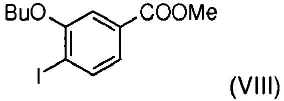

Etape 4 : Le composé (VIII) est ensuite réduit à l'aide de borohydrure de lithium pour donner le (3-Butoxy-4-iodo-phenyl)-methanol (IX)

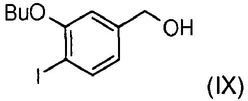

Etape 5 : L'oxydation du (3-Butoxy-4-iodo-phenyl)-methanol (IX) à l'aide de dioxyde de manganese permet d'obtenir le 3-butoxy-4-iodo-benzaldehyde (X).

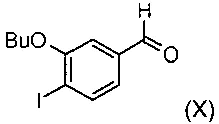

Etape 6 : La réaction de Wittig entre le 3-butoxy-4-iodo-benzaldehyde (X) et le (diéthoxy-phosphoryl)-méthoxy-acétate de méthyle (XI) en présence d'hydrure de sodium permet d'obtenir un mélange de (Z)-3-(3-Butoxy-4-iodo-phényl)-2-méthoxy-acrylate de méthyle (II) et de (E)-3-(3-Butoxy-4-iodo-phényl)-2-méthoxy-acrylate de méthyle (II') dans un ratio 60/40. Le (Z)-3-(3-Butoxy-4-iodo-phényl)-2-méthoxy-acrylate de méthyle (II) est obtenu pur après chromatographie sur gel de silice.

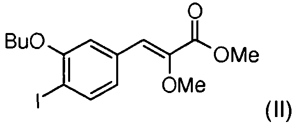

Etape 7 : Une réaction de couplage au palladium de type suzuki est réalisée entre le (Z)-3-(3-Butoxy-4-iodo-phényl)-2-méthoxy-acrylate de méthyle (II) et l'acide 3-(1-méthyl-3-pentyl-ureido)-phényl-boronique (III) pour donner le (Z)-3-[2-Butoxy-3'-(3-heptyl-1-methylureido)-biphenyl-4-yl]-2-methoxy-acrylate de méthyle (IV)

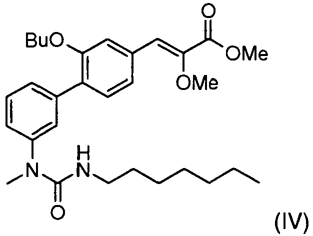

Etape 8 : Une réaction de saponification du (Z)-3-[2-Butoxy-3'-(3-heptyl-1-methylureido)-biphenyl-4-yl]-2-methoxy-acrylate de méthyle (IV) à l'aide hydroxyde de sodium permet d'obtenir l'acide (Z)-3-[2-Butoxy-3'-(3-heptyl-1-methyl-ureido)-biphenyl-4-yl]-2-methoxy-acrylique (I)

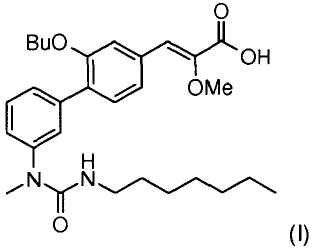

Un premier inconvénient de cette voie de synthèse est l'utilisation d'une séquence réactionnelle linéaire, longue, en 8 étapes, entre le composé commercial (V) et l'acide (Z)-3-[2-Butoxy-3'-(3-heptyl-1-methyl-ureido)-biphenyl-4-yl]-2-methoxy-acrylique (I). Une synthèse linéaire est toujours défavorable à une application à une échelle industrielle pour laquelle on préfèrera une synthèse convergente notamment en raison de la recherche d'un meilleur rendement.

D'autre part, l'utilisation de dioxyde de manganèse présente également un inconvénient au niveau des rejets industriels qu'il génère.

Enfin, la séparation des deux isomères (Z) et (E) du 3-(3-Butoxy-4-iodo-phényl)-2-méthoxy-acrylate de méthyle à l'étape 6 mettant en oeuvre une technique de chromatographie sur gel de silice est difficilement transposable dans un procédé industriel.

De plus, le (Z)-3-(3-Butoxy-4-iodo-phényl)-2-méthoxy-acrylate de méthyle (II) intermédiaire dans ce procédé est une huile incolore, ce qui peut présenter des problèmes d'isolation pour la transposition à une échelle industrielle.

Enfin, la purification de l'acide (Z)-3-[2-Butoxy-3'-(3-heptyl-1-methyl-ureido)-biphenyl-4-yl]-2-methoxy-acrylique (I) dans la voie de synthèse précédemment décrite nécessite l'utilisation de trois chromatographies sur gel de silice.

La présente invention vise donc à résoudre les problèmes cités ci-dessus en proposant un procédé de synthèse de l'acide (Z)-3-[2-Butoxy-3'-(3-heptyl-1-methyl-ureido)-biphenyl-4-yl]-2-methoxy-acrylique de formule (I) simplifié, comportant moins d'étapes, plus économique et susceptible d'être adapté en synthèse industrielle.

En effet, le nouveau procédé de synthèse de l'acide (Z)-3-[2-Butoxy-3'-(3-heptyl-1-methyl-ureido)-biphenyl-4-yl]-2-methoxy-acrylique (I), objet de la présente invention, est une synthèse convergente comprenant au maximum une séquence linéaire de 4 étapes comme présenté à la figure 2.

Ce nouveau procédé permet également de préparer en 3 étapes l'acide (Z)-3-(3-Butoxy-4-trifluoromethanesulfonyloxy-phenyl)-2-methoxy-acrylique de formule (XIIa) comme intermédaire réactionnel dans la préparation de l'acide (Z)-3-[2-Butoxy-3'-(3-heptyl-1-methylureido)-biphenyl-4-yl]-2-methoxy-acrylique (I).

Les étapes du procédé selon l'invention sont les suivantes :

### Etape 1 du procédé de l'invention :

Dans une première étape, le nouveau procédé de synthèse fait intervenir une réaction de Wittig entre l'halogénure de (Methoxy-alkoxycarbonyl-methyl)-triphenylphosphonium) (XVII) [*préparé selon le procédé décrit dans* Tetrahedron 1994, 50, 7543-7556*.]* et le 3-butoxy-4-hydroxy-benzaldehyde (XV), [*préparé par réaction de couplage entre le 3-bromo-4-hydroxy-benzaldehyde (XVI) commercial et un butanoate de metal alcalin comme le sodium par exemple en présence de sel de cuivre (I) ou d'un complexe de palladium dans un solvant aprotique polaire comme le diméthylformamide (DMF) par exemple à une température comprise entre 60°C et 150°C (La publication* Synth Commun 1990, 20, 2659 *décrit des réactions similaires*)] en présence d'une amine organique comme la triéthylamine par exemple dans un solvant aprotique comme le tetrahydrofurane (THF) par exemple permet d'obtenir le (Z)-3-(3-Butoxy-4-hydroxy-phenyl)-2-methoxy-acrylate d'alkyle de formule générale (XIV) où R1 représente un radical alkyle. Cette procédure permet d'obtenir spécifiquement l'isomère (Z) (>95%), ce qui permet, contrairement à la voie de synthèse décrite dans la demande internationale WO2007/049158, de s'affranchir de toute opération de séparation d'isomères Z/E sur gel de silice.

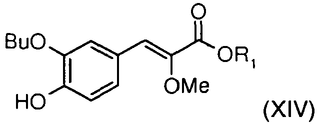

### Etape 2 du procédé de l'invention :

Le (Z)-3-(3-Butoxy-4-hydroxy-phenyl)-2-methoxy-acrylate d'alkyle (XIV) est mis en réaction avec de l'anhydride triflique par exemple ou du chlorure de mesyle ou du chlorure de benzenesulfonyle ou du chlorure de tosyle en présence d'une base organique comme par exemple la triéthylamine, dans un solvant comme le dichloromethane pour donner respectivement le (Z)-2-Methoxy-3-(3-butoxy-4-trifluoromethanesulfonyloxy-phenyl)-acrylate d'alkyle (Xllla) ou le (Z)-2-Methoxy-3-(3-butoxy-4-methanesulfonyloxy-phenyl)-acrylate d'alkyle (Xlllb) ou le (Z)-2-Methoxy-3-(3-butoxy-4-phenylsulfonyloxy-phenyl)-acrylate d'alkyle (Xlllc) ou le (Z)-2-Methoxy-3-(3-butoxy-4-tolylsulfonyloxy-phenyl)-acrylate d'alkyle (XIIId) :

| | | | |
|---|---|---|---|
| R1 = | radical alkyle | | 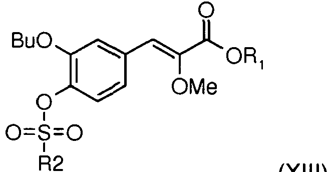 |
| R2 = | CF3 | (Xllla) | |
| | CH3 | (XIIIb) | |
| | Ph | (XIIIc) | |
| | 4-Tolyl | (XIIId) | (XIII) |

### Etape 3 du procédé de l'invention :

La saponification de l'ester (XIII), par exemple, du (Z)-2-Methoxy-3-(3-butoxy-4-trifluoromethanesulfonyloxy-phenyl)-acrylate d'alkyle (XIIIa) à l'aide d'une base comme l'hydroxyde de lithium par exemple dans un solvant comme le THF par exemple en présence d'eau permet d'obtenir l'acide (Z)-3-(3-Butoxy-4-trifluoromethanesulfonyloxy-phenyl)-2-methoxy-acrylique (XIIa).

De la même façon, la saponification à partir des esters (XIIb), (XIIIc) ou (XIIId) conduit respectivement aux acides (Xllb), (Xllc) ou (XIIId).

| | | | |
|---|---|---|---|
| | | | 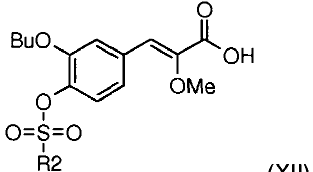 |
| R2 = | CF3 | (XIIa) | |
| | CH3 | (XIIb) | |
| | Ph | (XIIc) | |
| | 4-Tolyl | (XIId) | (XII) |

### Etape 4 du procédé de l'invention :

L'étape 4 du procédé objet de la présente invention consiste en la synthèse de l'acide (Z)-3-[2-Butoxy-3'-(3-heptyl-1-methyl-ureido)-biphenyl-4-yl]-2-methoxy-acrylique de formule (I) par réaction de couplage, de préférence catalysée au palladium ou au nickel, entre, par exemple, l'acide (Z)-3-(3-butoxy-4-trifluoromethanesulfonyloxy-phenyl)-2-methoxy-acrylique (XIIa) et la 3-Heptyl-1-methyl-1-[3-(4,4,5,5-tetraméthyl-[1,3,2]-dioxaborolan-2-yl)-phenyl]-urée (IIIb) 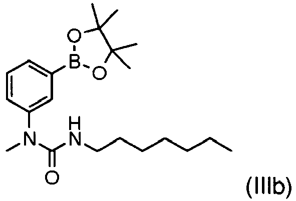 ou son acide boronique correspondant (IIIa)

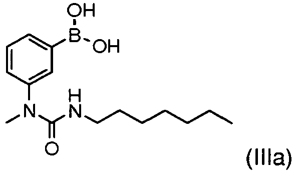

Le composé (IIIb) peut être préparé notamment comme décrit dans la demande internationale WO2007/049158, à savoir:

### a- 1-(3-Bromo-phényl)-3-heptyl-1-méthyl-urée :

On prépare la (3-bromo-phényl)-méthylamine comme suit :

150g (500 mmoles) de (3-bromo-phényl) -*N*-méthylcarbamate de tert-butyle sont placés dans 600ml de dichlorométhane et 383ml (5 moles) d'acide trifluoroacétique. Le milieu réactionnel est agité à température ambiante pendant 24 heures, puis traité avec une solution aqueuse saturée de carbonate de sodium et extrait au dichlorométhane. La phase organique est lavée à l'eau, séchée sur sulfate de magnésium, filtrée et évaporée. 99g (100%) de (3-bromo-phényl)-méthyl-amine sont obtenus.

Puis :

3,2ml (20 mmoles) d'isocyanate d'heptyle sont ajoutés sur une solution de 2,5g (13 mmoles) de (3-bromo-phényl)-méthylamine ainsi préparée dans 10ml de tétrahydrofuranne en présence de 2ml de triéthylamine. Le mélange réactionnel est agité 12 heures à température ambiante. La réaction est arrêtée par l'ajout de 2ml d'eau puis extraite avec de l'acétate d'éthyle. Les phases organiques sont rassemblées et séchées sur sulfate de sodium. Les solvants sont évaporés puis le résidu est purifié par chromatographie sur colonne de silice élué avec un mélange heptane/acétate d'éthyle 70/30. 3,4g (77%) de 1-(3-bromo-phenyl)-3-heptyl-1-methyl-urée sous forme de solide sont obtenus.

### b- 3-Heptyl-1-méthyl-1-[3-(4,4,5,5-tétraméthyl-[1,3,2]dioxaborolan-2-yl)-phényl)-urée :

4,0g (15,5 mmoles) de bis-pinacol diboranne sont ajoutés sur un mélange de 3,4 g (10 mmoles) de 1-(3-bromo-phényl)-3-heptyl-1-méthyl-urée, 3,0g (31 mmoles) d'acétate de potassium, en présence de 380mg (0,5 mmole) de dichlorure de palladium diphénylphosphino-ferrocène dans 15ml de diméthylformamide. Le mélange est agité 3 heures à 90°C. La réaction est arrêtée par l'ajout de 50ml d'eau puis extraite avec de l'acétate d'éthyle. Les phases organiques sont rassemblées et séchées sur sulfate de sodium. Les solvants sont évaporés puis le résidu est purifié par chromatographie sur colonne de silice élué avec un mélange heptane/acétate d'éthyle 70/30. 2,5g (64%) de 3-heptyl-1-méthyl-1-[3-(4,4,5,5-tétraméthyl-[1,3,2]dioxaborolan-2-yl)-phényl]-urée (IIIb) sont obtenus sous la forme d'huile.

Le composé (IIIa) est quant à lui préparé également comme décrit dans la demande internationale WO2007/049158, à savoir:

### a- 1-(3-Bromo-phényl)-3-heptyl-1-méthyl-urée :

### Mode opératoire identique à celui décrit ci-dessus.

### b- acide 3-(3-heptyl-1-méthyl-ureido)-phényl-boronique :

Par réaction de 113g (345 mmoles) de 1-(3-bromo-phényl)-3-heptyl-1-méthyl-urée dans 1,1l de tétrahydrofurane, de 127ml (380 mmoles) d'une solution de méthyllithium 1,6M dans l'éther diéthylique, de 530ml (760 mmoles) d'une solution de tert-butyllithium 1,7M dans le pentane et de 97ml (904 mmoles) de triméthyle borane, 36g (36%) d'acide 3-(3-heptyl-1-méthyl-ureido)-phényl-boronique (IIIa) sous la forme d'une poudre rosée sont obtenus après purification du résidu brut par chromatographie sur gel de silice élué avec un mélange heptane/acétate d'éthyle 50/50 et cristallisation dans un mélange acétate d'éthyle/heptane.

De la même façon, la réaction de couplage à partir des acides (Xllb), (Xllc) ou , (XIId) (acides de formule (XII) pour lesquels R2 représente un groupement méthyle, un groupement phényle ou un groupement tolyle) conduit à l'acide (Z)-3-[2-Butoxy-3'-(3-heptyl-1-methyl-ureido)-biphenyl-4-yl]-2-methoxy-acrylique de formule (I).

L'acide (Z)-3-[2-Butoxy-3'-(3-heptyl-1-methyl-ureido)-biphenyl-4-yl]-2-methoxy-acrylique de formule (I) est purifié par recristallisation dans du diisopropyl éther, technique transposable à l'échelle industrielle.

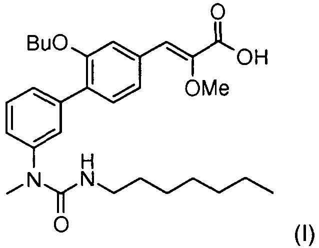

Les conditions de couplage de cette dernière étape sont bien connues de l'homme du métier. Ce dernier, dans le cadre de la présente invention, peut aussi bien utiliser des conditions classiques de couplage *(voir* A. Suzuki et al., Synth. Commun. 1981, 11, 513 *ou* Sharp. M.J. Tet, Lett. 1985, 26, 5997*)* que des conditions optimisées *(voir par exemple* Littke. A.F. et al, J Am Chem Soc 2000, 122 (17), 4020-4028*).* Le mode de mise en oeuvre décrit ci-après implique un choix non exclusif de conditions.

Selon la présente invention, on désigne par alkyle une chaîne hydrocarbonée saturée, linéaire ou ramifiée, comprenant de 1 à 4 atomes de carbone. De préférence, un tel radical est choisi parmi les radicaux méthyle, éthyle, propyle, butyle, i-propyle et t-butyle.

Selon la présente invention, on désigne par alkoxy un atome d'oxygène substitué par une chaîne hydrocarbonée saturée, linéaire ou ramifiée, comprenant de 1 à 4 atomes de carbone.

Selon la présente invention, on désigne par alkoxycarbonyle un carbonyl substitué par un alkoxy tel que défini précédemment.

Selon la présente invention, on désigne par halogène un atome de chlore, de fluor, de brome ou d'iode.

La présente invention concerne également les différents intermédiaires réactionnels mis en oeuvre dans le procédé de l'invention, à savoir:
- les sulfonates de formule générale (XII) où R2 est choisi parmi un groupement méthyle, un groupement trifluorométhyle, un groupement phényle et un groupement tolyle, représentés par l'acide (Z)-3-(3-Butoxy-4-trifluoromethanesulfonyloxy-phenyl)-2-methoxy-acrylique (Xlla), l'acide (Z)-3-(3-Butoxy-4-methanesulfonyloxy-phenyl)-2-methoxy-acrylique (Xllb), l'acide (Z)-3-(3-Butoxy-4-phenylsulfonyloxy-phenyl)-2-methoxy-acrylique (Xllc) et l'acide (Z)-3-(3-Butoxy-4-tolylsulfonyloxy-phenyl)-2-methoxy-acrylique (XIId)

| | | | |
|---|---|---|---|
| | | | 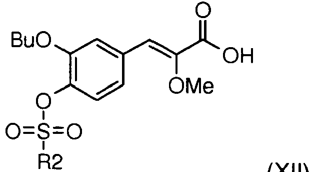 |
| R2 = | CF3 | (Xlla) | |
| | CH3 | (Xllb) | |
| | Ph | (XIIc) | |
| | 4-Tolyl | (Xlld) | (Xll) |

- les sulfonates de formule générale (XIII) dans laquelle R1 représente un radical alkyle et R2 représente un groupement méthyle, un groupement trifluorométhyle, un groupement phényle ou un groupement tolyle, et représentés par le (Z)-2-Methoxy-3-(3-butoxy-4-trifluoromethanesulfonyloxy-phenyl)-acrylate d'alkyle (Xllla), le (Z)-2-Methoxy-3-(3-butoxy-4-methanesulfonyloxy-phenyl)-acrylate d'alkyle (Xlllb), le (Z)-2-Methoxy-3-(3-butoxy-4-phenylsulfonyloxy-phenyl)-acrylate d'alkyle (XIIIc) et le (Z)-2-Methoxy-3-(3-butoxy-4-tolylsulfonyloxy-phenyl)-acrylate d'alkyle (XIIId)

| | | | |
|---|---|---|---|
| R1 = | radical alkyle | | 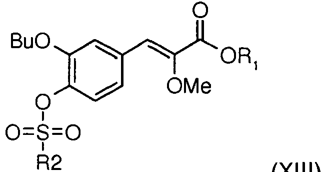 |
| R2 = | CF3 | (XIIIa) | |
| | CH3 | (XIIIb) | |
| | Ph | (XIIIc) | |
| | 4-Tolyl | (Xllld) | (XIII) |

- le (Z)-3-(3-Butoxy-4-hydroxy-phenyl)-2-methoxy-acrylate d'alkyle (XIV) où R1 est un radical alkyle:

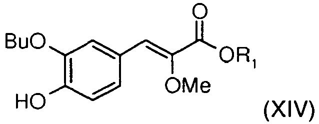

D'autres caractéristiques et avantages de la présente invention apparaîtront encore à la lecture du mode opératoire ci-après donné à titre illustratif et non limitatif.

### PREPARATION DES MATIERES PREMIERES

### 3-butoxy-4-hydroxy-benzaldehyde (XV)

2.16 g (93.9 mmol) de sodium sont dissous dans 15 mL de n-butanol à 110°C pendant 3 heures. 20 mL de diméthylformamide sont ajoutés à température ambiante puis le milieu est dégazé plusieurs fois. 3.4 g (34.1 mmol) de chlorure de cuivre I sont ajoutés à température ambiante puis le milieu réactionnel est agité pendant 10 minutes. 6.2 g (31 mmol) de 3-bromo-4-hydroxy-benzaldehyde sont ajoutés puis le milieu réactionnel est agité 2 heures à 120°C. La réaction est refroidie à température ambiante puis arrêtée par l'ajout de 50 mL d'une solution d'acide chlorhydrique 2M. Le milieu est extrait avec 250 mL d'acétate d'éthyle. Les solvants sont évaporés puis le résidu est filtré sur un patch de gel de silice (2 cm) : éluant (Heptane/Acétate d'éthyle 8/2). Après évaporation des solvants, 5.55 g d'huile orangée sont obtenus. Cette huile est cristallisée avec du pentane. 4.8 g de 3-butoxy-4-hydroxy-benzaldehyde sous forme de solide brun sont obtenus après filtration. Rendement = 80%.

RMN ¹H (400 MHz, CDCl₃) : 0.99 (t, *J* = 8 Hz, 3H, C*H*₃) ; 1.52 (hex, *J* = 8 Hz, 2H, C*H*₂) ; 1.85 (pent, *J* = 8 Hz, 2H, C*H*₂) ; 4.14 (t, *J* = 8 Hz, 2H, C*H*₂) ; 6.24 (s, 1H, ArH) ; 7.05 (d, *J* = 8 Hz, 1 H, ArH) ; 7.42 (m, 2H, ArH + OH) ; 9.83 (s, 1H, C*H*O).

### Chlorure de (Methoxy-methoxycarbonyl-methyl)-triphenyl-phosohonium (XVII)

25 g (0.186 mol) de diméthoxyacétate de methyle sont ajoutés à 27 mL (0.215 mol) de chlorure d'acétyle à température ambiante. 0.1 g (0.2 mol %) de di-iode sont ajoutés puis le mélange réactionnel est agité 16 heures à 55°C. l'excès de chlorure d'acétyle est évaporé sous vide puis le résidu est dissous dans 100 mL de dichlorométhane. 49 g (0.204 mol) de triphénylphosphine sont ajoutés puis le mélange réactionnel est agité à 37°C pendant 3 heures. Les solvants sont évaporés puis le résidu est cristallisé dans de l'éther diisopropylique. 70 g de chlorure de (Methoxy-methoxycarbonyl-methyl)-triphenylphosphonium sont obtenus.
Rendement = 88%.

RMN ¹H (400 MHz, CDCl₃) 3.61 (s, 3H, OC*H*₃) ; 3.90 (s, 3H, OC*H*₃) ; 7.66 (m, 6 H, ArH), 7.77 (m, 3H, ArH) ; 8.01 (m, 6H, ArH) ; 8.71 (d, *J* = 13Hz, 1H, C*H*).

### ETAPES DU PROCEDE

### ETAPE 1 : (Z)-3-(3-Butoxy-4-hvdroxy-oheny/)-2-methoxy-acrylate de méthyle (XIV)

4.0 g (20.6 mmol) de 3-butoxy-4-hydroxy-benzaldehyde et 13.25 g (30.9 mmol) de chlorure de (Methoxy-methoxycarbonyl-methyl)-triphenyl-phosphonium sont mis en suspension dans 40 mL de tetrahydrofurane. 6 mL (42 mmol) de triéthylamine sont ajoutés puis le milieu réactionnel est agité 16 heures à 40°C. Le milieu réactionnel est filtré, puis les solvants sont évaporés. 50 mL de diethyl éther sont ajoutés, le mélange est agité 10 minutes à température ambiante puis filtré. Après évaporation des solvants, le résidu est repris avec un mélange heptane/acétate d'éthyle 8/2 puis filtré sur un patch de silice (2 cm). 5.3 g de (Z)-3-(3-Butoxy-4-hydroxy-phenyl)-2-methoxy-acrylate de méthyle sont obtenus après évaporation des solvants.
Rendement = 92%.

RMN ¹H (400 MHz, CDCl₃) : 0.91 (t, *J* = 8 hz, 3H, C*H*₃) ; 1.42 (hex, *J* = 8 Hz, 2H, C*H*₂) ; 1.75 (pent, *J* = 8Hz, 2H, C*H*₂) ; 3.66 (s, 3H, OC*H*₃) ; 3.78 (s, 3H, OC*H*₃) ; 4.00 (t, *J* = 8 Hz, 2H, C*H*₂) 5.79 (s, 1H, C*H*=) ; 6.84 (d, *J* = 8 Hz, 1H, ArH), 6.88 (s, 1H, ArH) ; 7.13 (d, *J* = 8 Hz, 1H, ArH) ; 7.39 (s, 1H, O*H*).

### ETAPE 2: (Z)-2-Methoxy-3-(3-butoxy-4-trifluoromethanesulfonyloxy-phenyl)-acrylate de méthyle (XIIIa).

2.6 g (9.27 mmol) de (Z)-3-(3-Butoxy-4-hydroxy-phenyl)-2-methoxy-acrylate de méthyle sont dissous dans 25 mL de dichloromethane puis 2 mL de triéthylamine sont ajoutés. 1.67 mL d'anhydride triflique (10.2 mmol) sont ajoutés lentement à 0°C. Le mélange réactionnel est agité 2 heures à 0°C. La réaction est arrêtée à l'aide d'une solution saturée d'hydrogénocarbonate de sodium. Le milieu est extrait avec de l'acétate d'éthyle. Les phases organiques sont rassemblées, séchées sur sulfate de sodium puis filtrées sur un patch de silice (1 cm) les solvants sont évaporés. 3.57 g de (Z)-2-Methoxy-3-(3-butoxy-4-trifluoromethanesulfonyloxy-phenyl)-acrylate de méthyle sont obtenus.
Rendement = 93%.
RMN ¹H (400 MHZ, CDCl₃) : 0.91 (t, *J* = 7 hz, 3N, C*H*₃) ; 1.46 (hex, *J* = 7 Hz, 2H, C*H*₂) ; 1.76 (pent, *J* = 7Hz, 2H, C*H*₂) ; 3.73 (s, 3H, OC*H*₃) ; 3.80 (s, 3H, OC*H*₃) ; 4.00 (t, *J* = 7 Hz, 2H, C*H*₂) ; 6.83 (s, 1H, C*H*=) ; 7.11 (d, *J* = 8 Hz, 1 H, ArH) ; 7.20 (d, *J* = 8 Hz, 1 H, ArH) ; 7.45 (s, 1 H, ArH)

### ETAPE 3: Acide (Z)-3-(3-Butoxy-4-trifluoromethanesulfonyloxy-phenyl)-2-methoxy acrylique (XIIa)

1.53 g (36.3 mmol) d'hydroxyde de lithium monohydraté sont ajoutés sur une solution de 7.5 g (18.2 mmol) de (Z)-2-Methoxy-3-(3-butoxy-4-trifluoromethanesulfonyloxy-phenyl)-acrylate de méthyle dans 50 mL d'un mélange 10/1 de tetrahydrofuranne/eau. Le mélange réactionnel est agité 5 heures à 68°C. Le milieu réactionnel est acidifié avec une solution d'acide chlorhydrique 2N jusqu'à pH =1. Le mélange réactionnel est extrait avec deux fois 100 mL d'un mélange Heptane/Acétate d'éthyle 1/2. Les phases organiques sont rassemblées puis sechées sur sulfate de sodium. Les solvants sont évaporés puis le résidu est repris dans 30 mL de pentane. 4.7 g d'acide (Z)-3-(3-Butoxy-4-trifluoromethanesulfonyloxy-phenyl)-2-methoxy-acrylique sont obtenus sous la forme d'un solide blanc.
Rendement = 63%.

RMN H (400 MHz, CDCl₃) : 1.00 (t, *J* = 7 hz, 3H, C*H*₃) ; 1.56 (hex, *J* = 7 Hz, 2H, C*H*₂) ; 1.86 (pent, *J* = 7Hz, 2H, C*H*₂) ; 3.86 (s, 3H, OC*H*₃) ; 4.10 (t, *J* = 7 Hz, 2H, C*H*₂) ; 7.07 (s, 1H, C*H*=) ; 7.23 (d, *J* = 8 Hz, 1 H, ArH) ; 7.33 (d, *J* = 8 Hz, 1H, ArH) ; 7.56 (s, 1H, ArH)

### ETAPE 4: Acide (Z)-3-[2-Butoxy-3'-(3-heptyl-7-methyl-ureido)-biphenyl-4-yl]-2-methoxy-acrytique (I)

870 mg (2.2 mmol) d'acide (Z)-3-(3-Butoxy-4-trifluoromethanesulfonyloxy-phenyl)-2-methoxy-acrylique (XII), 981 mg (2.62 mmol) de (3-Heptyl-1-methyl-1-[3-(4,4,5,5-tetramethyl-[1,3,2]dioxaborolan-2-yl)-phenyl]-urée (III) [préparé selon la demande internationale WO2007/049158], 14 mg (3 mol%) d'acétate de palladium, et 46 mg (6 mol%) de dicyclohexylbiphenylphosphine sont dissous dans 8 mL de diméthylformamide, 1.5 mL d'une solution 2 M de phosphate de potassium sont ajoutés et le milieu est dégazé plusieurs fois. Le mélange réactionnel est agité 2 heures à 90°C. La réaction est arrêtée avec une solution d'acide chlorhydrique 2M puis le mélange est extrait avec de l'acétate d'éthyle. Les phases organiques sont lavées avec une solution de chlorure de sodium puis secnees sur Na₂SO₄. Les solvants sont évaporés puis le résidu est repris dans 10 mL d'heptane/AcOEt 1/1. On filtre sur un patch de silice (1 cm) Evaporation des solvants. Le résidu est précipité dans du pentane. Le solide est recristallisé dans de l'éther isopropylique/heptane. On obtient 510 mg d'acide (Z)-3-[2-Butoxy-3'-(3-heptyl-1-methyl-ureido)-biphenyl-4-yl]-2-methoxy-acrylique sous forme de solide.
Rendement = 47%.
Point de fusion = 99 °C

RMN ¹H (400 MHz, CDCl₃) : 0,85 (t, *J* = 7Hz, 3H, C*H*₃); 0,95 (t, *J* = 7 Hz, 3H, C*H*₃); 1,24 (m, 8H, C*H*₂); 1,42 (m, 4H, C*H*₂); 1,78 (m, 2H, C*H*₂); 3,18 (td, *J* = 7 Hz, *J* = 6Hz, 2H, NC*H*₂); 3,33 (s, 3H, NC*H*₃); 3,89 (s, 3H, OC*H*₃); 4,05 (t, *J* = 7 Hz, 2H, OC*H*₂); 4,46 (t, *J* = 6 Hz, 1H, NH); 7,17 (s, 1H, =C*H*); 7,23 (d, *J* = 8 Hz, 1H, Ar*H*); 7,35-7,54 (m, 6H, Ar*H*).

## Revendications

1. Procédé de synthèse de l'acide (Z)-3-[2-Butoxy-3'-(3-heptyl-1-methyl-ureido)-biphenyl-4-yl]-2-methoxy-acrylique de formule (I) 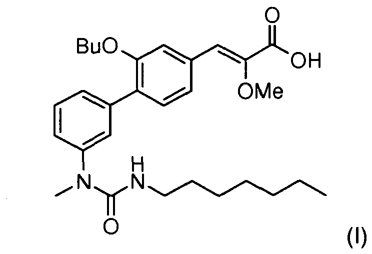 **caractérisé en ce qu'**on fait réagir un sulfonate de formule générale (XII) 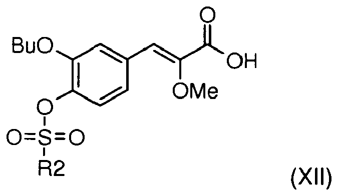 dans laquelle R2 représente un groupement methyle, un groupement trifluoromethyle, un groupement phenyle ou un groupement tolyle avec le 3-Heptyl-1-methyl-1-[3-(4,4,5,5-tetramethyl-[1,3,2]dioxaborolan-2-yl)-phenyl]-urée de formule (IIIb) 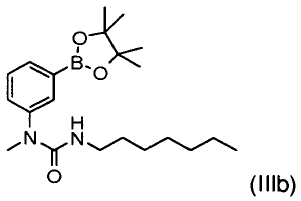 ou son acide boronique correspondant de formule (IIIa)

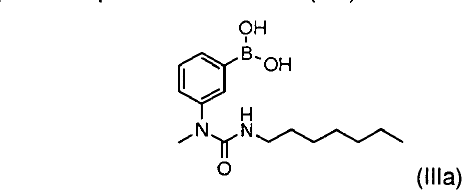

2. Procédé selon la revendication 1, **caractérisé en ce que** la réaction est effectuée en présence d'un catalyseur au palladium ou au nickel.

3. Procédé selon la revendication 1 ou 2, **caractérisé en ce que** le sulfonate de formule
générale (XII) 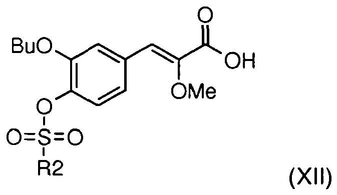 est obtenu par saponification de l'ester de formule générale (XIII). 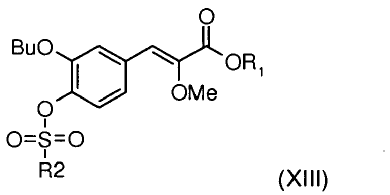 dans laquelle R1 représente un radical alkyle.

4. Procédé selon la revendication 3, **caractérisé en ce que** l'ester de formule générale (XIII) 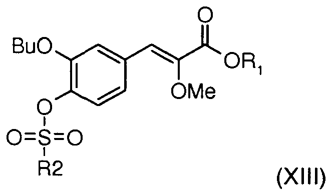 est obtenu par réaction du (Z)-3-(3-Butoxy-4-hydroxy-phenyl)-2-methoxy-acrylate d'alkyle de formule générale (XIV) 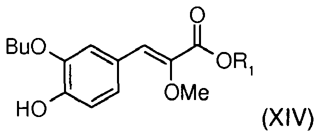 avec un composé choisi parmi l'anhydride triflique, le chlorure de mesyle, le chlorure de benzenesulfonyle et le chlorure de tosyle.

5. Procédé selon la revendication 4, **caractérisé en ce que** le (Z)-3-(3-Butoxy-4-hydroxyphenyl)-2-methoxy-acrylate d'alkyle de formule (XIV) 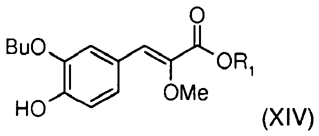 est obtenu par réaction de Wittig entre le 3-butoxy-4-hydroxy-benzaldehyde de formule (XV) 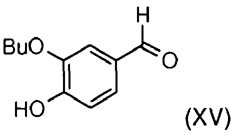 et un halogénure de (Methoxy-alkoxycarbonyl-methyl)-triphenyl-phosphonium) de formule (XVII).

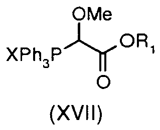

6. Composé de formule générale (XII) 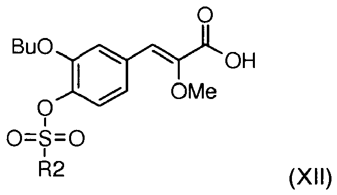 dans laquelle R2 représente un groupement methyle, un groupement trifluoromethyle, un groupement phenyle ou un groupement tolyle

7. Composé de formule générale (XIII) 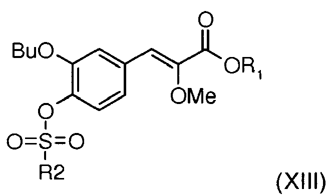 dans laquelle R1 représente un radical alkyle et R2 représente un groupement methyle, un groupement trifluoromethyle, un groupement phenyle ou un groupement tolyle.

8. Composé (Z)-3-(3-Butoxy-4-hydroxy-phenyl)-2-methoxy-acrylate d'alkyle de formule générale (XIV) 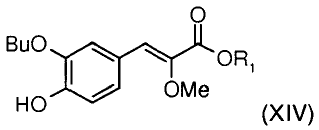 dans laquelle R1 représente un radical alkyle.

## Claims

1. Process for the synthesis of (Z)-3-[2-butoxy-3'-(3-heptyl-l-methylureido)biphenyl-4-yl]-2-methoxyacrylic acid of formula (I) 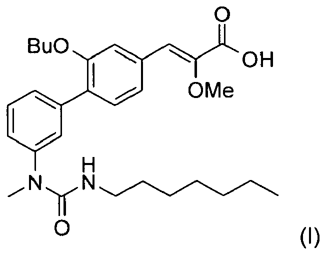 **characterized in that** a sulphonate of general formula (XII) : 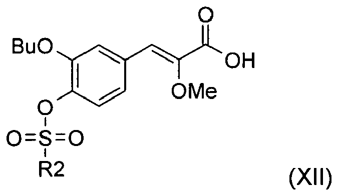 in which R2 represents a methyl group, a trifluoromethyl group, a phenyl group or a tolyl group, is reacted with 3-heptyl-1-methyl-1-[3-(4,4,5,5-tetramethyl-1,3,2-dioxoborolan-2-yl)phenyl]urea of formula (IIIb) 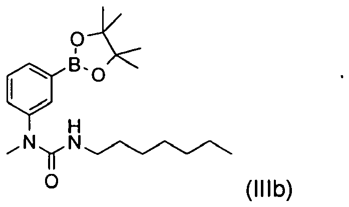 or its corresponding boronic acid of formula (IIIa)

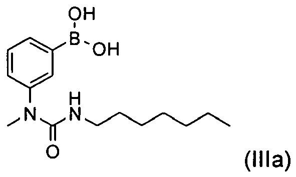

2. Process according to Claim 1, **characterized in that** the reaction is carried out in the presence of a palladium or nickel catalyst.

3. Process according to Claim 1 or 2, **characterized in that** the sulphonate of general formula (XII) 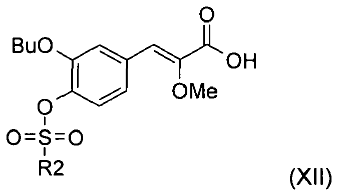 is obtained by saponification of the ester of general formula (XIII): 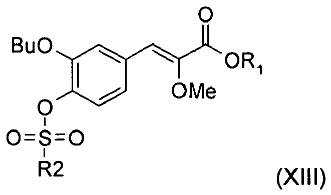 in which R₁ represents an alkyl radical.

4. Process according to Claim 3, **characterized in that** the ester of general formula (XIII): 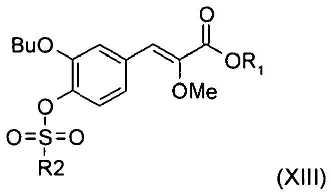 is obtained by reaction of alkyl (Z)-3-(3-butoxy-4-hydroxyphenyl)-2-methoxyacrylate of general formula (XIV) : 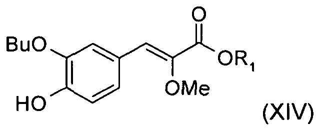 with a compound chosen from triflic anhydride, mesyl chloride, benzenesulphonyl chloride and tosyl chloride.

5. Process according to Claim 4, **characterized in that** alkyl (Z)-3-(3-butoxy-4-hydroxyphenyl)-2-methoxyacrylate of formula (XIV): 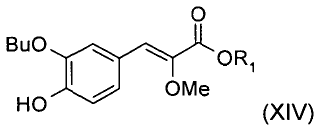 is obtained by a Wittig reaction between 3-butoxy-4-hydroxybenzaldehyde of formula (XV): 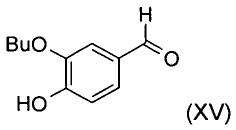 and a (methoxy(alkoxycarbonyl)methyl)triphenylphosphonium halide of formula (XVII):

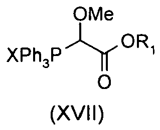

6. Compound of general formula (XII): 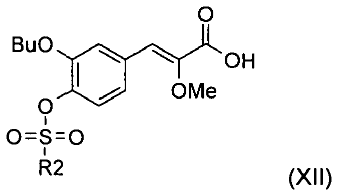 in which R2 represents a methyl group, a trifluoromethyl group, a phenyl group or a tolyl group.

7. Compound of general formula (XIII): 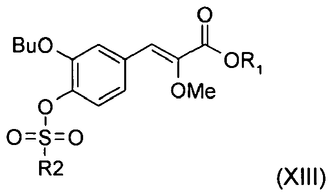 in which R₁ represents an alkyl radical and R2 represents a methyl group, a trifluoromethyl group, a phenyl group or a tolyl group.

8. Compound alkyl (Z)-3-(3-butoxy-4-hydroxyphenyl)-2-methoxyacrylate of general formula (XIV) : 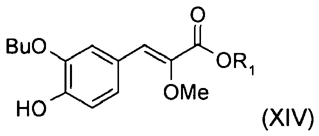 in which R₁ represents an alkyl radical.

## Patentansprüche

1. Verfahren zur Synthese von (Z)-3-[2-Butoxy-3'-(3-heptyl-1-methylureido)biphenyl-4-yl]-2-methoxyacrylsäure der Formel (I) 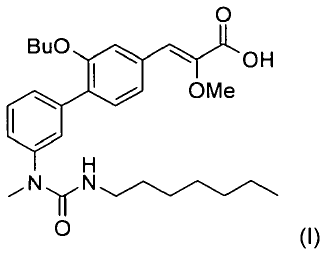 **dadurch gekennzeichnet, dass** man ein Sulfonat der allgemeinen Formel (XII) 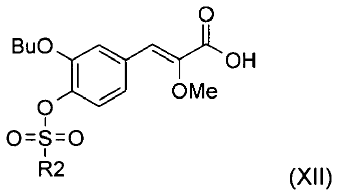 worin R2 für eine Methylgruppe, eine Trifluormethylgruppe, eine Phenylgruppe oder eine Tolylgruppe steht, mit 3-Heptyl-1-methyl-1-[3-(4,4,5,5-tetramethyl[1,3,2]dioxoborolan-2-yl)phenyl]harnstoff der Formel (IIIb) 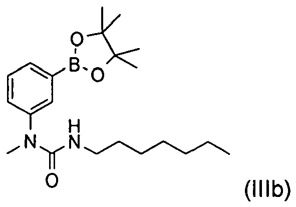 oder der entsprechenden Boronsäure der Formel (IIIa) 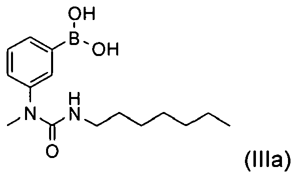 umsetzt.

2. Verfahren nach Anspruch 1, **dadurch gekennzeichnet, dass** man die Umsetzung in Gegenwart eines Palladium- oder Nickelkatalysators durchführt.

3. Verfahren nach Anspruch 1 oder 2, **dadurch gekennzeichnet, dass** man das Sulfonat der allgemeinen Formel (XII) 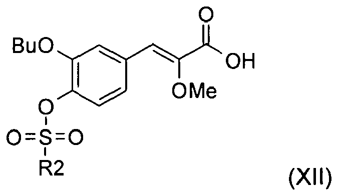 durch Verseifung des Esters der allgemeinen Formel (XIII) 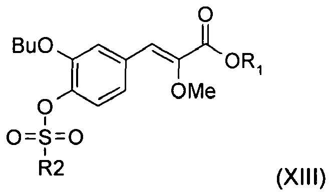 worin R1 für einen Alkylrest steht, erhält.

4. Verfahren nach Anspruch 3, **dadurch gekennzeichnet,**
**dass** man den Ester der allgemeinen Formel (XIII) 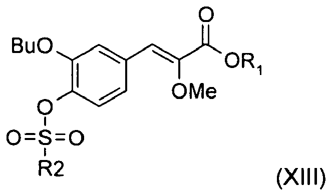 durch Umsetzung eines (Z)-3-(3-Butoxy-4-hydroxyphenyl)-2-methoxyacrylsäurealkylesters der allgemeinen Formel (XIV) 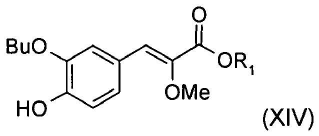 mit einer unter Trifluormethansulfonsäureanhydrid, Mesylchlorid, Benzolsulfonylchlorid und Tosylchlorid ausgewählten Verbindung erhält.

5. Verfahren nach Anspruch 4, **dadurch gekennzeichnet, dass** man den (Z)-3-(3-Butoxy-4-hydroxyphenyl)-2-methoxyacrylsäurealkylester der allgemeinen Formel (XIV) 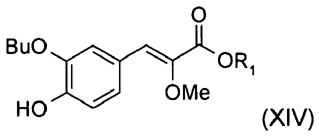 durch Wittig-Reaktion zwischen 3-Butoxy-4-hydroxybenzaldehyd der Formel (XV) 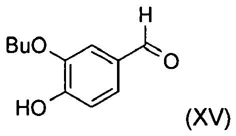 und einem (Methoxy-alkoxycarbonyl-methyl)tri-phenylphosphonium)halogenid der Formel (XVII) 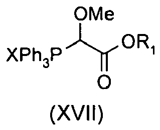 erhält.

6. Verbindung der allgemeinen Formel (XII) 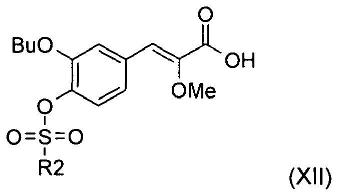 worin R2 für eine Methylgruppe, eine Trifluormethylgruppe, eine Phenylgruppe oder eine Tolylgruppe steht.

7. Verbindung der allgemeinen Formel (XIII) 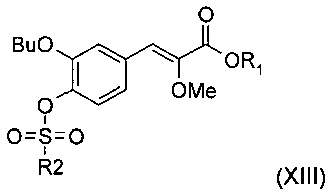 worin R1 für einen Alkylrest steht und R2 für eine Methylgruppe, eine Trifluormethylgruppe, eine Phenylgruppe oder eine Tolylgruppe steht.

8. Verbindung (Z)-3-(3-Butoxy-4-hydroxyphenyl)-2-methoxyacrylsäurealkylester der allgemeinen Formel (XIV) 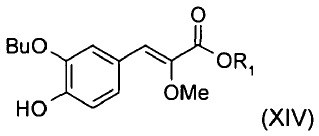 worin R1 für einen Alkylrest steht.
